(19) Europäisches Patentamt
European Patent Office
Office européen des brevets

(11) EP 2 340 797 A1

(12) # EUROPEAN PATENT APPLICATION

(43) Date of publication:
**06.07.2011 Bulletin 2011/27**

(21) Application number: **10252138.2**

(22) Date of filing: **17.12.2010**

(51) Int Cl.:
***A61F 15/00*** *(2006.01)* ***A47F 1/12*** *(2006.01)*
***A61K 8/25*** *(2006.01)* ***A61K 8/58*** *(2006.01)*
***A61K 8/895*** *(2006.01)* ***B65D 83/04*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.12.2009 US 287815 P**
**18.12.2009 US 287810 P**

(71) Applicant: **McNeil-PPC, Inc.**
**Skillman, NJ 08558-9418 (US)**

(72) Inventors:
- **Bissah, Kofi A.**
  **Somerset, New Jersey 08873 (US)**
- **de Oliveira, Ricardo**
  **New Hope, Pennsylvania 18938 (US)**
- **Desai, Saurabh**
  **Somerset, New Jersey 08873 (US)**
- **Fung, Paul Y.**
  **South River, New Jersey 08882 (US)**
- **Librizzi, Joseph J.**
  **Hillsborough, New Jersey 08844 (US)**
- **Nguyen, Thong**
  **Vestal, New York 13850 (US)**
- **Pillai, Shoba**
  **Feasterville, Pennsylvania 19053 (US)**

(74) Representative: **Kirsch, Susan Edith**
**Carpmaels & Ransford**
**One Southampton Row**
**London**
**WC1B 5HA (GB)**

(54) **Product dispenser and absorbent article kit**

(57) A product dispenser assembly for dispensing a liquid or gel composition, the assembly including a container housing having a head portion and an internal chamber for holding a composition, the head portion including at least one and preferably a plurality of apertures, preferably a moveable member structured to enable a user to selectively deploy the composition from within the internal chamber through at least one aperture, a cap assembly, a first absorbent pad arranged on top of the head portion for receiving the composition as it is deployed from the container, a plurality of absorbent pads arranged in a stacked configuration within cap assembly, each of the plurality of pads being adapted to be sequentially arranged on top of the head portion after the first removable pad has used to apply the composition and then removed by the user. Kits comprising the product dispenser assembly and at least one absorbent article are also disclosed.

EP 2 340 797 A1

## Description

### FIELD OF THE INVENTION

The present invention relates to a product dispenser for dispensing a personal care composition, and in particular for a product dispenser for dispensing and applying such a composition to a surface of the human body. The dispenser according to the present invention is particularly useful for applying such a composition to the intimate area of the body. The present invention also relates to compositions for repelling fluid from a surface, in particular a surface of the human body. The compositions of the present invention comprise a volatile carrier, a powder-feel agent, and an ester, and are useful, for example, in those areas of the human body which are prone to wetness, such as the intimate area. The compositions of the present invention further tend to aid in preventing odor, skin irritation and chafing associated with exposure to moisture or fluid. The present invention further relates to a kit including a product dispenser of the type described above in combination with an absorbent article.

### BACKGROUND OF THE INVENTION

The intimate area and other surfaces of the body which come in regular contact with fluid can be a source of discomfort and embarrassment in both men and women. Such discomfort and embarrassment may be associated, for example, with adults that suffer from incontinence, and the regular monthly cycles of women during their reproductive years. In both these cases, the discomfort is generally related to irritation and the feeling of wetness and the embarrassment is usually due to the presence of odor. Also, in children, especially infants, the intimate area can become irritated due to the contact of urine and feces with the skin.

To overcome these issues, products have been developed which either absorb fluids or wick the fluid away from the body. Examples of such types of products include sanitary protection articles, diapers and incontinence products. Over the years, several improvements have been made to such products to aid in absorbing or wicking fluid, for example, superabsorbent material has been added to the constructions, new materials have been developed for the cover layer, and transfer layers added to help wick the fluid into the absorbent layers. Additionally, odor control agents have been incorporated to absorb or mask the odor. Inclusion of fragrances may also add additional odor control. All these improvements are based on an external absorbent product which wicks away the fluid or moisture.

Additionally, people for years have used products such as zinc oxide, oil or petrolatum to repel fluid from their skin. These products, while performing quite well at providing a water-proof barrier also left an undesirable sticky feel to the skin.

Examples of other compositions designed to overcome the sticky feel can be found in US 6200964 and US 6384023 (both to Singleton et al.) and US 20060159645 (Miller et al.). These references all use a volatile liquid and a silicone polymer.

Nevertheless, applicants have recognized the need for compositions that are more effective at repelling fluid from a surface, such as the surface of a human body than prior compositions, and preferably overcome the sticky feel associated with prior compositions as well. In addition, applicants have recognized the need for a product dispenser that can be used effectively dispense and apply such compositions to the body in a discreet, sanitary and simple fashion.

### SUMMARY OF THE INVENTION

In view of the foregoing the present invention provides, according to a first aspect of the invention, a product dispenser assembly comprising:

a container housing including an internal chamber for holding a composition and a head portion, the head portion including at least one aperture, the container housing being structured and arranged to enable a user to selectively deploy the composition from within the internal chamber through the at least one aperture;

a cap assembly structured and arranged to be placed on top of the head portion of the container, the cap assembly including an outer shell having a first open end and a second closed end;

a first removable absorbent pad arranged on top of the head portion for receiving the composition as it is deployed from the container, the first removable pad being adapted to enable a user to apply the composition to the body and manually remove the pad from the head portion after the composition has been applied;

a plurality of absorbent pads arranged in a stacked configuration within the outer shell, each of the plurality of pads being adapted to be sequentially arranged on top of the head portion after the first removable pad has been used to apply the composition and then removed from the head portion by the user.

The head portion of the product dispenser assembly may comprise a plurality of apertures.

The container housing may comprise a moveable member arranged within the container housing that is selectively moveable by a user within the container to allow a user to selectively manipulate a volume of the internal chamber, the moveable member being adapted to enable a user to selectively deploy the composition from within the internal chamber, through the at least one aperture, by reducing the volume within the chamber.

The invention also provides a product dispenser assembly comprising:

a container housing having a head portion and an internal chamber for holding a composition, the head portion including a plurality of apertures;
a moveable member arranged within the container housing that is selectively moveable by a user within the container to allow a user to selectively manipulate a volume of the internal chamber, the moveable member being adapted to enable a user to selectively deploy the composition from within the internal chamber, through the apertures, by reducing the volume within the chamber;
a cap assembly structured and arranged to be placed on top of the head portion of the container, the cap assembly including an outer shell having a first open end and a second closed end;
a first absorbent pad arranged on top of the head portion for receiving the composition as it is deployed from the container, the first removable pad being adapted to enable a user to apply the composition to the body and manually remove the pad from the head portion after the composition has been applied;
a plurality of absorbent pads arranged in a stacked configuration within the outer shell, each of the plurality of pads being adapted to be sequentially arranged on top of the head portion after the first removable pad has been used to apply the composition and then removed from the head portion by the user.

The invention further provides a kit including a product dispenser assembly of the present invention and at least one absorbent article.

## BRIEF DESCRIPTION OF THE DRAWINGS

FIG. 1 is a perspective view of a product dispenser assembly according to a first embodiment the present invention;
FIG. 2 is a partially exploded view of the product dispenser assembly shown in FIG. 1 showing the cap assembly removed from the container housing;
FIG. 3 is a detailed perspective view of the cap assembly showing the manner in which a plurality of absorbent pads are arranged within the outer shell of the cap assembly;
FIG. 4 is a sectional view of the cap assembly taken along line 4-4 in FIG. 2;
FIG. 5 is a partially exploded perspective view showing the manner in which one of the absorbent pads is arranged on the head portion of the container housing;
FIG. 5a is a detailed perspective view of the head portion, according to one embodiment of the invention, showing the encircled region of head portion in FIG. 5;
FIG. 6 is a sectional view of the container housing and absorbent pad taken along line 6-6 in FIG. 2;
FIG. 7 is a detailed sectional view of container housing and absorbent pad, showing the encircled portion in FIG. 6, and depicting the manner in which the composition contained within the container housing permeates through the absorbent pad during use of the assembly;
FIG. 8 and 9 are partial sectional views of the cap assembly and container housing showing the manner in which a clean pad is retained on the head portion of container housing when the cap assembly is removed from the container housing;
FIG. 10 is a perspective view of an absorbent pad and container housing according to an alternate embodiment of the present invention;
FIG. 11 is a detailed sectional view taken along line 11-11 in FIG. 10 and depicting the manner in which the composition contained within the container housing passes through the apertures of the absorbent pad;
FIG. 12-14 are perspective views of a product dispenser assembly according to a second embodiment the present invention, depicting the manner in which an absorbent pad can be manually removed from the cap assembly and applied to the head portion of the container housing.

## DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference.

All percentages listed in this specification are percentages by weight, unless otherwise specifically mentioned.

As used herein, the term "intimate area" shall mean the area near or between the thighs, including the crotch area of a human where body exudates, such as urine, feces, vaginal discharge, menstrual fluid, and the like, may be present. The intimate area shall also include the breasts. The intimate area is typically covered by undergarments or absorbent articles.

As used herein the term "absorbent articles" includes articles such as diapers (infant and adult), sanitary napkins, shields, pantyliners, breast pads, and the like.

Referring now to the drawings, a product dispenser assembly 10 according to a first embodiment of the invention is shown in Figs. 1-11. As seen in Fig. 1, the product dispenser assembly 10, generally includes a container housing 12 and a removable cap assembly 14 to protect the composition contained within the housing 12.

As best seen in Fig. 6, the container housing 12 defines an internal chamber 16 adapted to hold a composition 18. Preferably the composition 18 is in the form of a liquid or gel. The container housing 12 further includes a head portion 20 that includes a plurality of apertures 22 as best seen in Figs. 6 and 7. As described in further detail below, the plurality of apertures 22 permit a user to selectively deploy the composition from within the internal chamber 16 through the apertures 22.

As seen in Fig. 6, the product dispenser assembly 10 further includes a moveable member 24 arranged within the container housing 12 and adapted to enable a user to selectively manipulate a volume of the internal chamber 16. By manipulating the volume of the internal chamber 16, and specifically by reducing the volume of the internal chamber, a user can selectively deploy the composition 18 through the apertures 22. A central portion of the movable member 24 is provided with a threaded coupling sleeve 26 for cooperation with an elevator screw 28. The lower end of the elevator screw 28 is axially fixed but rotatable within an opening 29 of the container housing 12. The lower end of elevator screw 28 is operatively coupled to a knob 30. Rotation of the knob 30 permits the user to raise or lower the moveable member 24 relative to the container housing 12. In this manner, the user can manually control the volume within the internal chamber 16 and thereby deploy the composition 18 from within the chamber 16 through the apertures 22.

As best seen in Fig. 2 and Fig. 5, the product dispenser assembly 10 further includes a first absorbent pad 32 that is arranged on top of the head portion 20 of the container housing 12. As shown in Fig. 7, the first absorbent pad 32 is adapted to receive the composition 18 as it is deployed from the internal chamber 16 and through the apertures 22. As will be described in greater detail below, the absorbent pad 32 is adapted temporarily absorb and retain the composition to enable a user to apply the composition 18 to the body of the user by placing the pad 32 in contact with the body.

As seen in Fig. 5a, a top surface 34 of the head portion 20 may be provided with a material including a plurality of hooks 36 adapted to retain the absorbent pad 32 in place while the user applies the composition 18 to the body. The material including the plurality of hooks 36 also permits a user to manually remove the absorbent pad 32 from the head portion 20, and dispose of the same, after the pad 32 has been used to apply the composition 18 to the body. Particularly suitable hook materials of the type referred to above are commercially available from Velcro USA Inc., Manchester, New Hampshire. Other means may also be used to retain the pad absorbent pad 32 in place, such as certain adhesives, provided that such means permit the user to selectively remove the pad 32 after it has been used to apply the composition 18 to the body.

As shown in Fig. 1 the cap assembly 14 is structured and arranged to be placed on top of the head portion 20 of the container housing 12. As shown in Figs. 3 and 4, the cap assembly 14 includes an outer shell 38 that has a first open end 40 and a second opposed closed end 42. As shown in Fig. 3, the outer shell 38 is structured and arranged to hold a plurality of absorbent pads 44 that are arranged in a stacked configuration within the outer shell 38. As shown in Fig. 4, the plurality of pads 44 includes a lowermost pad 44a that is arranged closest to the open end 40 of the outer shell 38 and a plurality of pads 44b arranged on top of the lowermost pad 44a. As shown in Figs. 3 and 4, the outer shell 38 includes a rib structure 46 that extends outwardly from an internal surface 48 of the outer shell 38. As will be described in greater detail below, the rib structure 46 is adapted to retain the plurality of pads 44 within the cap assembly 14 until such time as each of the pads 44 is ready for use.

As shown in Fig. 4, the cap assembly 14 further includes a platform 48 arranged within the outer shell 38. As will be described in greater detail below, the platform 48 is adapted to urge the plurality of absorbent pads 44 toward the open end 40 of the outer shell 38. The cap assembly 14 further includes a resilient member 50 that is arranged between the outer shell 38 and the platform 48 and is adapted to urge the platform 48 towards the open end 40 of the outer shell 38. The resilient member 50 may comprise, for example, a spring having a first end coupled to the outer shell 38 and a second end coupled to the platform 48. Other suitable resilient member structures may alternatively be employed provided that they effectively urge the platform 46 towards the open end 40 of the outer shell 38.

The first absorbent pad 32 and each of the plurality of absorbent pads 44 are preferably formed from a fibrous

material. Suitable fibrous materials include, without limitation, woven, nonwoven (oriented, e.g., via a carding process, or non-oriented), or knit fabrics. The fibers may be integrated into a nonwoven structure via, for example, needle punching, through-air bonding, hydro entangling, spun-bonding, chemical bonding (including adhesive bonding), or mechanical processing (such as embossing).

The first absorbent pad 32 and each of the plurality of absorbent pads 44 preferably has a thickness of about 0.5 mm to about 5 mm, more preferably from about 1 mm to about 5 mm. Preferably each absorbent pad has a basis between about 10 gsm (g/$m^2$) and about 450 gsm, preferably between about 300 and about 400 gsm. The fibrous material forming each absorbent pad preferably includes rayon to provide softness and a strong, resilient material such as an olefin or polyester. One particularly suitable fibrous material is a needle-punched blend of staple-length 1.5 denier "TENCEL" rayon and staple-length 4-5 denier PET available from Precision Custom Coating, Totowa, NJ.

The container housing 12, removable cap assembly 14, moveable member 24, elevator screw 28, knob 30, as well as the constituent parts thereof, can be constructed from any suitable rigid material. Preferably the container housing 12, removable cap assembly 14, moveable member 24, elevator screw 28, knob 30, as well as the constituent parts thereof, are formed from plastic by molding, although other suitable rigid materials could be used.

Referring to Figs. 10 and 11, the absorbent pad 32 may optionally be provided with a plurality of holes 33 intended to promote the flow of the composition through and into the absorbent pad 32 as shown in Fig. 11. In the specific embodiment of the invention shown in Figs. 10 and 11 the holes 33 are arranged such that they correspond in location to the apertures 22 in the container housing 12. Of course, it is not required that the plurality of holes 33 and apertures 22 correspond exactly in number and/or location, provided that the holes 33 and apertures 22 promote the flow of the composition through and into the absorbent pad 32.

The method of using the product dispenser assembly 10 according to the first embodiment of the invention will now be described with reference to Figs. 1-9. First the user removes the cap assembly 14 from the container housing 12 as shown in Figs. 1 and 2. Once the cap assembly 14 is removed from the container housing 12 the first absorbent pad 32 is revealed as shown in Fig. 2. Thereafter, the user can turn the knob 30 (Fig. 6) thereby causing the moveable member 24 to move in an upward direction thereby reducing the volume of the internal chamber 16. By reducing the volume of the internal chamber 16, the composition 18 is urged through the apertures 22 in the head portion 20 of the container housing 12, as shown in Fig. 7. As the composition 18 is urged through the apertures 22 such composition is then absorbed by the first absorbent pad 32. Thereafter, the user may use the product dispenser assembly 10 to apply the composition to the body by placing the first absorbent pad 32 in surface to surface contact with the body. The first absorbent pad 32 is retained in place during application of the composition 18 to the body by means of the material including the plurality of hooks 36 that is arranged on the top surface 34 of the head portion 20, as seen in Fig. 5 and 5a. After the first absorbent pad 32 is used to apply the composition 18 to the body, the user may manually remove the first absorbent pad 32 from the head portion 20 and discard of the same. Thereafter, the user places the cap assembly 14 back on the container housing 12 as shown in Fig. 8. Once the cap assembly 14 is placed back on the container housing 12, the lowermost pad 44a of the plurality of pads 44 is placed in abutment with the top surface 34 of the head portion, and therefore in abutment with the material including the plurality of hooks 36, and the plurality of hooks 36 engage lowermost pad 44a. Once the user is ready to use the product dispenser assembly 10 again, the user removes the cap assembly 14, as shown in Fig. 9. Upon removal of the cap assembly 14, the lowermost pad 44a is retained in place on the top surface 34 of the head portion 20 by the material including the plurality of hooks 36. In this manner the lowermost pad 44a is automatically applied to, and retained on, the head portion 20 of the container housing 12. As shown in Fig. 9, as the cap assembly 14 is removed from the container housing 12, the platform 48 is urged toward the open end 40 of the outer shell 38 by the resilient member 50. In this way the remainder of the plurality of absorbent pads 44b are urged towards the open end 40 of the outer shell 38. The absorbent pads 44b are retained within the cap assembly 14 by the rib structure 46. In this manner, a new clean absorbent pad 44 is always ready for use when the user removes the cap assembly 14.

A product dispenser assembly 10a according to a second embodiment of the invention is shown in Figs. 12-14. The product dispenser assembly 10a according to second embodiment is substantially identical to the product dispenser assembly 10b described above. However, the product dispenser assembly 10a does not include the platform 48 or the resilient member 50. Rather, when the user desires to replace the used first absorbent pad 32, the user simply manually removes a new clean absorbent pad 44 from the cap assembly 14, as shown in Fig. 13 and manually applies the clean absorbent pad 44 to the head portion 20 of the container housing 12 as shown in Fig. 14. To facilitate the manual removal of the clean absorbent pad 44 from cap assembly 14, the outer shell 38 is provided with an access port 52 as shown in Figs. 12 and 13. The product dispenser assembly 10a is in all other respects identical to the product dispenser 10 according to the first embodiment as described above.

The product dispenser assemblies described herein are preferred embodiments of the present invention, however other embodiments are possible within the scope and spirit of the claimed invention. For example, the container housing could be constructed as a flexible tube (e.g. like a toothpaste tube) to enable a user to manually deploy the composition from within the tube by squeezing the tube. Other embodiments of the invention, within the spirit and scope

of the claimed invention, will be apparent to those of skill in the art based upon the disclosure of the invention made herein.

**Personal Care Compositions**

Personal care compositions particularly suitable for use with the dispenser assembly described above are described in detail below. Although preferred compositions are described in detail below, other liquid or gel compositions could be used with the dispensing device described above.

The composition of the present invention contains at least three components: a volatile cyclic silicone, a silicone-based powder-feel agent and an ester selected from the group consisting of compounds of Formula I, Formula II, Formula III, and combinations of two or more thereof:

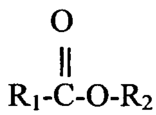
(I)

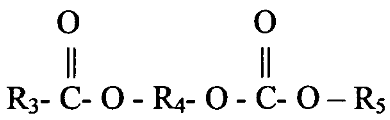
(II)

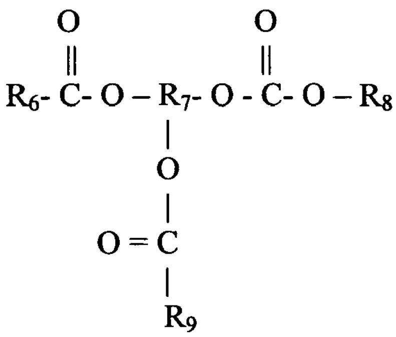
(III)

wherein $R_1$, $R_2$, $R_3$, $R_5$, $R_6$, $R_8$ and $R_9$ are independently linear or branched, substituted or unsubstituted, saturated or unsaturated, $C_3$-$C_{22}$ alkyl or alkenyl groups, $R_4$ is a linear or branched, substituted or unsubstituted, saturated or unsaturated, $C_3$-$C_{22}$ alkylene or alkenylene moiety, and $R_7$ is a linear or branched, substituted or unsubstituted, saturated or unsaturated, $C_3$-$C_{22}$ moiety, the composition being substantially anhydrous and the ester present in an amount of about 5% or less.

It has surprisingly been found that application of this composition results in a greater repulsion of fluid from the body than previously seen by other comparable compositions. This benefit is demonstrated by the measurement of the contact angle of water placed on a surface that has been treated with the composition in accord with the Contact Angle Test as described herein below. Applicants have discovered unexpectedly, that compositions of the present invention tend to exhibit a contact angle of 90° or greater. In certain preferred embodiments, the compositions exhibit a contact angle of 91° or greater and more preferably 92° or greater. In certain particularly preferred embodiments, the compositions exhibit a contact angle of 93° or greater.

Applicants have also measured the Body Dryness Index associated with compositions and uses of the present invention. Applicants have discovered that the claimed compositions provide a significant body dryness as compared to other comparable compositions.

Applicants have further recognized that in addition to unexpected fluid repellency, the compositions of the present invention may further be used on the body to deliver an aesthetically pleasing feel to the skin. Upon application to the skin, the composition delivers a "powdery" feel that is pleasing to the user and yet continues to deliver the benefit of lubrication and slip between the skin surface and other surfaces such as other skin surfaces or external clothing.

Applicants have also measured the Body Dryness Index associated with compositions and uses of the present invention. Applicants have discovered that the claimed compositions provide a significant body dryness as compared to other comparable compositions.

Any suitable volatile cyclic silicone carrier may be used in the present invention. As used herein the term "volatile" refers to those liquids that have a measurable vapor pressure at ambient temperature. Examples of suitable volatile cyclic silicone carriers include cyclomethicones of the formula:

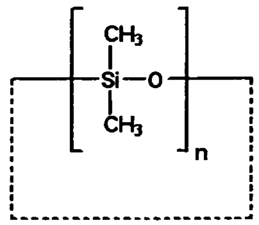

wherein n=3 to 6. Examples of certain preferred volatile cyclic silicone carriers include decamethylcyclopentasiloxane, dodecamethylcyclohexasiloxane, and the like. A particularly preferred volatile cyclic silicone is decamethylcyclopentasiloxane. A variety of commercially available volatile, cyclic polydimethylsiloxanes include: Dow Corning DC 244 and DC 344 fluids (cyclotetrasiloxanes) and DC 245 and DC345 (cyclopentasiloxanes) manufactured by Dow Coming, Midland Mich.; Volatile Silicone 7158, 7207 and 7349 manufactured by Momentive Performance Materials, Tarrytown, NY and KF9937 and KF9945 manufactured by Shin- Etsu Silicones.

Any material that is capable of delivering a "powdery" feel when released onto the skin may be used in the present invention as a powder-feel agent. Suitable powder-feel agents include a variety of silicone polymers, gels, gums, particulate materials, combinations of two or more thereof, and the like.

Examples of silicone polymers useful as powder-feel agents in the present invention include crosslinked siloxane (e.g., dimethicone or dimethicone derivatives) copolymers such as stearyl methyl-dimethyl siloxane copolymer (Gransil SR-CYC, available from Grant Industries, Elmwood Park, N.J.); dimethicone/vinyldimethicone crosspolymers; Polysilicone-11 (i.e., a crosslinked silicone rubber formed by the reaction of vinyl terminated silicone and methylhydrodimethyl siloxane in the presence of cyclomethicone), cetearyl dimethicone/vinyl dimethicone crosspolymer (i.e., a copolymer of cetearyl dimethicone crosslinked with vinyl dimethyl polysiloxane), dimethicone/phenyl vinyl dimethicone crosspolymer (i.e., copolymer of dimethylpolysiloxane crosslinked with phenyl vinyl dimethylsiloxane), and dimethicone/vinyl dimethicone crosspolymer (i.e., copolymer of dimethylpolysiloxane crosslinked with vinyl dimethylsiloxane). More preferably, the compositions useful in the method of this invention include silicone elastomer blends containing dimethicone/vinyldimethicone crosspolymers (such as those made by Dow Corning), dimethicone, cyclopentasiloxane, trisiloxane, dimethicone and hydrophobically-modified silica.

The silicone polymers may be of any suitable molecular weight. In certain preferred embodiments, the polymers have a weight average molecular weight in excess of 10,000 (e.g., between about 10,000 and 10,000,000).

Examples of suitable silicone gels include the following which are also available commercial from Grant Industries by the indicated tradename: cyclomethicone (and) polysilicone-11 (Gransil GCM5), cyclotetrasiloxane(D4) (and) petrolatum (and) polysilicone-11 (Grangil PS-4), cyclopentasiloxane(D5) (and) petrolatum (and) polysilicone-11 (Gransil PS-5), cyclopentasiloxane(D5) (and) dimethicone (and) polysilicone-11 (Gransil DMCM-5), cyclotetrasiloxane(D4) (and) dimethicone (and) polysilicone-11 (Gransil DMCM-4), polysilicone-11 (and) isododecane (Gransil IDS), and cyclomethicone (and) polysilicone-11 (and) petrolatum (and) phytosphingosine (Gransil SPH). Other examples of such gels, available from General Electric, include cyclopentasiloxane (and) dimethicone/vinyl dimethicone crossploymer (SFE839). In general, the compositions set forth in U.S. Patent No. 6,200,964 and U.S. Patent No. 6,384,023, which are hereby incorporated herein by reference, are suitable for use in the methods of this invention.

Suitable silicone gels include silicone elastomer gels. The elastomer chemically is a crosslinked, 3-dimentional network of intertwined silicone polymers that swell in the presence of a carrier. Elastomers are not soluble in the carrier but swollen in the carrier. Typically the "effective" carrier solvent is a low molecular weight species that can migrate into the network. The crosslinking density of the elastomer can affect the "swelling" efficiency; generally, lower crosslinking density favors swelling (for a given carrier) and gives a "wetter" feel initially (sometimes could be sticky). Conversely a

higher crosslinking density elastomer swells less and gives a "drier" skin feel initially. Most elastomer products dry down to a "powdery" after-feel particularly if the solvent is volatile. One non-limiting example of a suitable class of silicone elastomers is crosslinked organopolysiloxane (or siloxane) elastomers, which are generally described in U.S. patent application publication US2003/0049212A1.

The crosslinked organopolysiloxane elastomers may be categorized as emulsifying or non-emulsifying. "Emulsifying," as used herein, means crosslinked organopolysiloxane elastomers having at least one polyoxyalkylene (e.g., polyoxyethylene or polyoxypropylene) or polyglycerin moiety. The polyoxyalkylene (e.g., polyoxyethylene or polyoxypropylene) or polyglycerin moiety may serve as the crosslinker within the elastomer. Exemplary emulsifying crosslinked organopolysiloxane elastomers are described in U.S. Pat. Nos. 5,412,004; 5,837,793, and 5,811,487. Suitable emulsifying crosslinked organopolysiloxane elastomers include dimethicone/PEG-10 crosspolymers such as KSG 24; dimethicone/PEG-10 crosspolymers such as KSG 21 and KSG 210; PEG-15/lauryl dimethicone crosspolymers such as KSG 31, KG 32, KSG 33, KSG 310, KG 320, KSG 330; PEG-15/lauryl dimethicone crosspolymers and PEG-10/lauryl dimethicone crosspolymers such as KSG 34 and KSG 340; dimethicone/polyglycerine-3 crosspolymers such as KSG-710; and lauryl dimethicone/polyglycerine-3 crosspolymers such as KSG 810, KSG 820, KSG 830, and KSG 840. Also from Shin-Etsu are Silicone Rubber powder, KMP-400 type, Silicone resin powder, KMP-590, X-52-1631, Hybrid silicone powders, KSP-100, KSP-101 and KSP-300, etc.

"Non-emulsifying" means crosslinked organopolysiloxane elastomers are essentially free of polyoxyalkylene or polyglycerin moieties. Exemplary non-emulsifying crosslinked siloxane elastomers include the CTFA (Cosmetic, Toiletry, and Fragrance Association International Cosmetic Ingredient Dictionary and Handbook, 11.sup.th ed.) designated dimethicone/vinyl dimethicone crosspolymers supplied by a variety of suppliers including Dow Corning (DC 9506), General Electric (SFE 839), Shin Etsu (KSG 15 and 16), and Grant Industries (GRANSIL RPS-NA) and dimethicone/ phenyl vinyl dimethicone crosspolymer such as KSG 18 available from Shin Etsu. Other exemplary non-emulsifying crosslinked siloxane elastomer include the CTFA designated dimethicone crosspolymers including Dow Corning. (DC 9040, DC 9041, DC 9045).

Also suitable are high molecular weight silicone gums with linear high molecular polymer "solids (gums)" which are soluble in a carrier and water-insoluble silicones inclusive of non-volatile polyalkyl and polyaryl siloxane gums and fluids, volatile cyclic and linear polyalkylsiloxanes, polyalkoxylated silicones, amino and quaternary ammonium modified silicones, rigid cross-linked and reinforced silicones and mixtures thereof. E.g, Dimethiconol (DC 2-9023 fluid), KF 8018 from shinetsu which is an amino gum in cyclomethicone.

The smooth soft, silky and powdery feel may also be achieved by using particulate materials. Typically, the particulate materials are free-flowing and solid (i.e., the particles are not hollow).

Suitable organic particulate materials include those available commercially under the tradenames as follows: those made ofpolymethylsilsesquioxane (e.g., Tospearl 145A available from GE Toshiba Silicone Co., Ltd.), polyamide (e.g., nylon-12 and Orgasol 2002D Nat C05 available from Atofina), polyolefines (e.g., Microthene FN510-00 available from Equistar), polyacrylates (e.g., ethylene acrylate copolymer, sold under the name FloBead EA209 available from Kobo), polymethacrylates (PMMA) (e.g., Micropearl M 100 available from Seppic), polystyrene (e.g., Dynospheres available from Dyno Particles), polytetrafluoroethylene (PTFE), polyurethanes, starch and starch derivatives, composite particles, and mixtures thereof. Copolymers derived from monomers of the aforementioned materials can also be used. The aforementioned polymers derived from carboxylic acid containing monomer further include ester and salts of the monomers.

Inorganic materials for improving skin feel include natural minerals such as mica, talc, and sericite, synthetic mica, synthetic sericite, plate-formed titanium oxide, plate-formed silica, plate-formed aluminum oxide, boron nitride, barium sulfate, plate-formed titania-silica composite oxide, and bismuth oxychloride. Further these inorganic particles comprising those described above as a base material and one or more inorganic oxides coating the base material such as titanium oxide, aluminum oxide, iron oxide, silicon dioxide, cerium oxide, and zirconium. The pure titanium or zinc oxides pigments may be coated with compounds such as amino acids such as lysine, silicones, lauroyl, collagen, polyethylene, lecithin and ester oils. The inorganic particles may be resin coated as cited in US patent application 2003/0171475. The resin is preferably one or more selected from the group consisting of polyurethane, a styrene-but adiene copolymer, an acrylonitrile-butadiene copolymer, a silicone-based elastomer, and a polyolefin-based elastomer.

According to certain preferred embodiments, the powder-feel agent is preferably a silicone-based powder feel agent such as a silicone polymer, silicone gels, silicone gums, hydrophobically-modified silica, combinations thereof, and the like. Preferred silicone-based powder feel agents include silicone elastomer blends and hydrophobic silica blends, combinations of two or more thereof, and the like.

Any suitable amounts of powder-feel agents may be used in the present invention. In certain embodiments, the powder feel agent is present in the composition in an amount of about 65% or less by weight of the total composition. In certain preferred embodiments, the powder feel agent is present in an amount of from about 5% to about 65%, more preferably from about 8% to about 65%, more preferably from about 8% to about 60%, more preferably from about 8% to about 40%, and even more preferably from about 8% to about 30% by weight of the total composition.

Suitable esters for use in the present invention include those of Formula I, Formula II, Formula III:

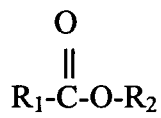 (I)

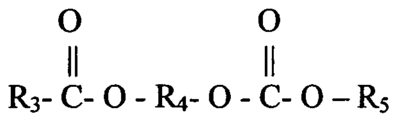 (II)

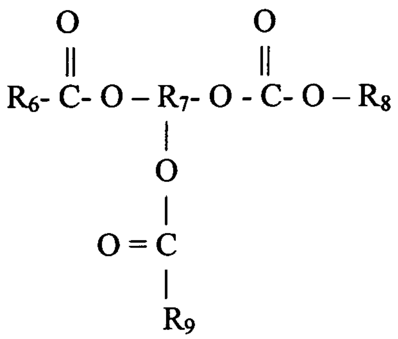 (III)

wherein $R_1$, $R_2$, $R_3$, $R_5$, $R_6$, $R_8$ and $R_9$ are independently linear or branched, substituted or unsubstituted, saturated or unsaturated, $C_3$-$C_{22}$ alkyl or alkenyl groups, $R_4$ is a linear or branched, substituted or unsubstituted, saturated or unsaturated, $C_3$-$C_{22}$ alkylene or alkenylene moiety, and $R_7$ is a linear or branched, substituted or unsubstituted, saturated or unsaturated, $C_3$-$C_{22}$ moiety. In certain preferred embodiments the ester of Formula I, Formula II, or Formula III have a viscosity ranging from about 10 to 1,000,000 centipoise at 25° C.

Examples of monoester oils of Formula I that may be used in the compositions of the invention include, but are not limited to, hexyldecyl benzoate, hexyl laurate, hexadecyl isostearate, hexydecyl laurate, hexyldecyl octanoate, hexyldecyl oleate, hexyldecyl palmitate, hexyldecyl stearate, hexyldodecyl salicylate, hexyl isostearate, butyl acetate, butyl isostearate, butyl oleate, butyl octyl oleate, cetyl palmitate, cetyl octanoate, cetyl laurate, cetyl lactate, octyl isononanoate, isostearyl isononanoate, isononyl isononanoate, cetyl isononanoate, cetyl stearate, stearyl lactate, stearyl octanoate, stearyl heptanoate, stearyl stearate, and so on.

Suitable diesters of Formula II that may be used in the compositions of the invention are the reaction product of a dicarboxylic acid and an aliphatic or aromatic alcohol, or a monocarboxylic acid and an aliphatic or aromatic alcohol containing at least two hydroxyl groups. Preferably, one or more of the acid or alcohol is a fatty acid or alcohol, i.e. contains 3-22 carbon atoms. The dicarboxylic acid may also be an alpha hydroxy acid. Examples of diester oils that may be used in the compositions of the invention include diisostearyl malate, neopentyl glycol dioctanoate, dibutyl sebacate, di-$C_{12}$-$_{13}$ alkyl malate, dicetearyl dimer dilinoleate, dicetyl adipate, diisocetyl adipate, diisononyl adipate, diisopropyl adipate, diisostearyl dimer dilinoleate, disostearyl fumarate, diisostearyl malate, isononyl isononanaote, isohexadecyl stearate, and so on.

Suitable triesters of Formula III comprise the reaction product of a tricarboxylic acid and an aliphatic or aromatic alcohol, or the reaction of an aliphatic or aromatic alcohol having three or more hydroxyl groups with mono-or dicarboxylic acids. Preferably, one or more of the acid or alcohol is a fatty acid or alcohol containing 3 to 22 carbon atoms. Examples of triesters include triarachidin, tributyl citrate, triisostearyl citrate, tri $C_{12}$-$_{13}$ alkyl citrate, tricaprylin, tricaprylyl citrate, tridecyl behenate, trioctyldodecyl citrate, tridecyl behenate, tridecyl cocoate, tridecyl isononanoate, and so on.

In certain preferred embodiments, the ester of the present invention is selected from the group consisting of

octyl isononanoate, isopropyl palmitate, butyl stearate, diisopropyl adipate, triisostearyl citrate, and combinations of two or more thereof. In certain more preferred embodiments, the ester is selected from the group consisting of octyl isononanoate, isopropyl palmitate, butyl stearate, and combinations of two or more thereof. In certain more preferred embodiments, the ester comprises octyl isononanoate.

The ester selected from the group consisting of esters of Formula I, Formula II, Formula III, and combinations of two or more thereof may be present in the compositions of the invention in an amount of 5% or less by weight based on the total weight of composition. In certain preferred embodiments, the ester is present in an amount of from about 0.5 to about 4.5%, more preferably from about 1 to about 4%, more preferably from about 2-4%, and even more preferably from about 2-3%.

In certain preferred embodiments, the composition is substantially anhydrous. As used herein, the term "substantially anhydrous" means that the composition contains less than 5% w/w water. In more preferred embodiments, the composition contains less than 3%, more preferably less than 2%, more preferably less than 1%, more preferably less than 0.5% w/w water. In certain preferred embodiments, the substantially anhydrous composition is an anhydrous composition (free of water).

The invention features a method of applying a cosmetic composition suitable for application to the skin, e.g., in the intimate area such as the perineum, under the breasts or on the thighs, of a subject in association with a cosmetically acceptable carrier. The individual components of the carrier are numerous and varied, but are also well known to one skilled in the art. In one aspect, the carrier comprises one or more of the members selected from the group consisting of acidifying agents, alkalizing agents, aerosol propellants, antimicrobial agents, antioxidants, buffering agents, chelating agents, coloring additives, dermatologically active agents, dispersing agents, emollients, emulsifying agents, humectants, fragrances, masking agents, preservatives, sugars, sunscreen agents, surfactants, suspending agents, thickening agents, an vehicles. These ingredients are discussed below. Examples of these agents are listed below as well as in the International Cosmetic Ingredient Dictionary and Handbook, eds. Wenninger and McEwen (The Cosmetic, Toiletry, and Fragrance Assoc., Washington, D.C., 7.sup.th Edition, 1997) (hereinafter "ICT Handbook").

Acidifying and alkalizing agents are preferably added to obtain the desired pH of the composition. Examples of acidifying agents included acetic acid, citric acid, glacial acetic acid, malic acid, and proprionic acid. Examples of alkalizing agent include edetol, potassium carbonate, potassium hydroxide, sodium borate, sodium carbonate, and sodium hydroxide. Other acidifying and alkalizing agents are listed on page 1653 of the ICT Handbook.

Aerosol propellants are used when the composition is to be administered as an aerosol under pressure. Examples of aerosol propellants include halogenated hydrocarbons such as dichlorodifluoromethane, dichlorotetrafluoroethane, and trichloromonofluoromethane, nitrogen, and volatile hydrocarbons such as butane, propane, isobutane, or mixtures thereof. Other propellants are listed on page 1655 of the ICT Handbook.

Anti-microbial agents are used when the area that the composition is to be applied is prone to microbial infection, e.g., by bacteria, fungal, or protozoa. Examples of such agents include benzyl alcohol, chlorobutanol, phenylethyl alcohol, phenylmercuric acetate, potassium sorbate, and sorbic acid, benzoic acid, butyl paraben, ethyl paraben, methyl paraben, propyl paraben, and sodium benzoate. Other anti-microbial agents are listed on page 1612 of the ICT Handbook.

Antioxidants are used to protect ingredients of the composition from oxidizing agents that are included within or come in contact with the composition. Examples of antioxidants include water soluble antioxidants such as ascorbic acid, sodium sulfite, metabisulfite, sodium bisulfite, sodium formaldehyde, sulfoxylate, isoascorbic acid, isoascorbic acid, cysteine hydrochloride, 1,4-diazobicyclo-(2,2,2)-octane, and mixtures thereof. Examples of oil-soluble antioxidants include ascorbyl palmitate, butytlated hydroxyanisole, butylated hydroxytoluene, potassium propyl gallate, octyl gallate, dodecyl gallate, phenyl-alpha-napthyl-amine, and tocopherols such as alpha-tocopherol. Other antioxidants are listed on pages 1612-13 of the ICT Handbook.

Coloring additives are used to add color to the composition. Examples of such coloring additives include titanium dioxide, yellow iron oxide, red iron oxide, black iron oxide, caramel, carmine, fluorescein derivatives, methoxsalen, trioxsalen, carbon black, azo dyes, anthraquinone dyes, blue azulenes, guajazulene, chamuzulene, erythrosin, bengal rose, phloxin, cyanosin, daphinin, eosin G, cosin 10B, and Acid Red 51. Other coloring agents are listed on pages 1628-30 of the ICT Handbook.

Dermatologically active agents include agents for treating wound healing, inflammation, acne, psoriasis, cutaneous aging, skin cancer, impetigo, herpes, chickenpox, dermatitis, pain, itching, and skin irritation. Examples of such dermatologically active agents include hydrocortisone, dexamethasone, panthenol, phenol, tetracycline hydrochloride, yeast, hexylresorcinol, lamin, kinetin, betamethagone, triamcinolone, fluocinolone, methylprednisolone, retinoids such as retinol and retinoic acid, dapsone, sulfasalazine, resorcinol, salicylic acid, benzoyl peroxide, erythromycin-benzoyl peroxide, erythromycin, clindamycin, mupirocin, griseofulvin, azoles such as miconazole, econazole, itraconazole, fluconazole, and ketoconazole, ciclopirox, allylamines such as naftifine and terfinafine, acyclovir, famciclovir, valacyclovir, benzocaine, lidocaine, dibucaine, pramoxine hydrochloride, methyl salicylate, camphor, menthol, resocinol, and vitamins such as tocopherol, tocopheryl acetate, pentothenic acid, ascorbic acid, biotin, and retinoids such as retinol, retinoic acid, retinal, retinyl acetate, and retinyl palmitate, alpha.-hydroxy acid, a .beta.-hydroxy acid, or poly-hydroxy acid such

as glycolic acid, lactic acid, citric acid, malic acid, and azaleic acid, and sunless tanning agents such as 1,3-dihydroxyacetone and 1,3,4-trihydroxy-2-butanone (erythulose).

Examples of dispersing and suspending agents include quarternium-18 hectorite, polyhydroxy stearic acid, poligeenan and silicon dioxide. Other dispersing and suspending agents are listed on page 1690-91 of the ICT Handbook.

Emollients are agents that soften and smooth the skin. Examples of emollients include hydrocarbon oils and waxes (e.g., natural and synthetic waxes) such as mineral oil, petrolatum, microcrystaline wax, polyethylene, triglyceride esters such as those of castor oil, cocoa butter, safflower oil, cottonseed oil, corn oil, olive oil, cod liver oil, almond oil, avocado oil, palm oil, sesame oil, squalene, and soybean oil, acetylated monoglycerides, ethoxylated glycerides, fatty acids, alkyl esters of fatty acids, alkenyl esters of fatty acids, fatty alcohols, fatty alcohol ethers, ether-esters, lanolin and derivatives of lanolin, polyhydric alcohol esters, wax esters such as beeswax, vegetable waxes, phospholipids, and sterols. Other emollients are listed on pages 1656-61 of the ICT Handbook.

Emulsifying agents are used for preparing emulsions of the present invention. Examples of emulsifying agents used for preparing silicone-in-oil, or oil-in-silicone emulsions include cyclomethicone (and) dimethicone copolyol, dimethicone copolyol, cetyl dimethicone copolyol,

Humectants are agents that promote the retention of moisture, e.g., moisturizers. Examples of humectants include sorbitol, matricaria extract, aloe barbadensis gel, glycerin, glycereth 5 lactate, glycereth 7 triacetate, glycereth 7 diisononoate, hexanetriol, hexylene glycol, propylene glycol, dipropylene glycol, alkoxylated glucose, D-panthenol, 1-2-pantandiol, 2-methyl-1,3-propanediol, and derivatives thereof, and hyaluronic acid. Other humectants are listed on pages 1661-62 of the ICT Handbook.

Examples of fragrances include peppermint, rose oil, rose water, aloe vera, clove oil, menthol, camphor, eucalyptus oil, and other plant extracts. Certain fragrances may require a solubilizer, e.g., PPG-5-ceteareth-20. To eliminate certain odors from compositions, masking agents may be used. An example of a masking agent includes ethylene brassylate. Other fragrances and masking agents are listed on pages 1639-40 of the ICT Handbook.

Preservatives are used to protect the composition from degradation. Examples of preservatives include liquipar oil, phenoxyethanol, methyl paraben, propyl paraben, butyl paraben, isopropyl paraben, isobutyl paraben, dieizolidinyl urea, imidazolidinyl urea, diazolindyl urea, benzalkonium chloride, benzethonium chloride, phenol, and mixtures thereof (e.g., liquipar oil). Other preservatives are listed on pages 1654-55 of the ICT Handbook.

Surfactants are agents used to stabilize multi-component compositions, e.g., used as wetting agents, antifoam agents, emulsifiers, dispersing agents, and penetrants. Examples of surfactants include methyl gluceth 20, decyl polyglucoside, lapyrium chloride, laureth 4, laureth 9, monoethanolamine, nonoxynol 4, nonoxynol 9, nonoxynol 10, nonoxynol 15, nonoxynol 30, poloxalene, polyoxyl 8, 40, and 50 stearate, polysorbate 20, polysorbate 40, polysorbate 60, polysorbate 65, polysorbate 80, and polysorbate 85, sodium lauryl sulfate, sorbitan and its derivatives. Other surfactants are listed on page 1672-90 of the ICT Handbook.

The cosmetically acceptable carrier that may be in a number of different delivery forms, e.g., a spray, mist, aerosol, mousse, semi-solid cream, liquid such as a solution, emulsion, or suspension, lotion, gel, solid such as a powder, adherent stick, flexible mask, self-hardening liquid or gel, or other suitable forms intended to be applied to the skin of a subject (e.g., a human).

The viscosity of the compositions of the present invention may be different dependent upon the type of formulation being prepared, e.g., a liquid formulation will have a lower viscosity than a gel or cream formulation. Typically, the viscosity of liquid formulations of the present invention will range from 5,000 to 25,000 cps. Bulking agents may be used to increase the viscosity of the composition.

The compositions of this invention may be prepared using methodology that is well known by an artisan of ordinary skill (e.g., by using well-known mixing and blending procedures).

## EXAMPLES

The following is a description of the manufacture of certain compositions of the present invention. Other compositions of the invention can be prepared in an analogous manner by a person of ordinary skill in the art.

### Test Procedure for Measuring Contact Angle (CAM)

Test fluid was made of the following mixture to simulate bodily fluids: 49.5% of 0.9% sodium chloride solution (VWR catalog # VW 3257-7), 49.05% Glycerin (Emery 917), 1% Phenoxyethanol (Clariant Corporation Phenoxetol.TM.) and 0.45% Sodium Chloride (Baker sodium chloride crystal # 9624-05).

For each sample, a 2 A 2" x 2" sample film was created using Leneta 2A Opacity charts, BYK Gardner Opacity Drawdown Base, and a BYK Gardner wet film drawdown bar with a thickness of 1.5mil. The Opacity Drawdown Base was used to hold the opacity charts at a constant position during the drawdown application process. The drawdown process involved placing small amount of gels across the top of the opacity charts and using the Drawdown bar to

vertically cover the chart with the gel. This vertically up-down motion was carried out until the gels formed a uniform film over the opacity charts. The charts were then cut into 2"x 2" film strips, and weighed on a Mettler Toledo scale.

Advancing contact angles of each sample strip were measured using the Krüss DS 100 Drop Shape Analysis machine. Following the manual instructions of the machine, the angle of inclination on were tilted to 2 degrees; the third syringe was selected; the drop type was changed to sessile drop; and the drop subtype was changed to normal sessile drop. The thickness of the needle was 0.509mm. Test fluid was placed in the machine and allowed to flush through the needle for a few minutes in order to remove flush the needle. The drop volume was manually controlled at a rate of 50μl/minute. With the camera in focus, contact angles of 4 sessile drops were measured on each strip. The sample size of this experiment is 4 (n=4) and Average Contact Angle Measurement (CAM) was recorded.

Comparative Example 1

Contact Angle Testing was performed to determine the contact angle of a series of esters (Repellant Agents) labeled as comparative compositions C1-C5.

Table 1

| Example | Ester/ Repellant Agent | INCI Name | Supplier | Location | Contact Angle Measurement |
|---|---|---|---|---|---|
| C1 | HallStar Octyl Isononanoate (Duck Oil) | Octyl isononanoate | **HallStar** Co | Hackettstown NJ, | 55.0° |
| C2 | HallStar IPP | Isopropyl palmitate | HallStar Co | Hackettstown, NJ | 53.1° |
| C3 | HallStar BST | Butyl stearate | HallStar Co | Hackettstown, NJ | 56.2° |
| C4 | Ceraphyl (DIPA) | Diisopropyl adipate | International Specialty Products | Wayne, NJ | 74.3° |
| C5 | TISC Ester | Triisostearyl citrate | Lubrizol Advanced Materials | Cleveland, OH | 78.6° |

Duck oil is a long chain monoester with two branches. IPP is a long chain monoester with a single chain. BST is a long chain monoester, unbranched. DIPA is a branched diester. TISC is a large, branched triester. All of these esters alone have a contact angle of less than 90°.

Example 1

A base formulation (B1), several inventive examples (E1-E5), and comparative examples (C6-C15) were prepared as described below. The CAM of each was measured and is reported below in Table 3.

A base formulation B1 was prepared using the ingredients of Table 2.

Table 2 - Base Formulation (B1)

| Function | Trade Name | INCI Name | Supplier | Address | Conc. w/w% |
|---|---|---|---|---|---|
| Volatilizing Agent | DC 245 Fluid | Decamethylcyclopentasiloxane | Dow Corning Corp. | Midland, MI | 36.57 |
| Powder Feel Agents | USG-103 | Dimethicone/vinyl dimethicone crosspolymer | Shin-Etsu Silicone of America | Akron, OH | 18.18 |
| Bulking Agents | DC 200 Fluid, 350 cSt | Dimethicone | Dow Corning Corp. | Midland, MI | 10.1 |

(continued)

| Function | Trade Name | INCI Name | Supplier | Address | Conc. w/w% |
|---|---|---|---|---|---|
| Bulking Agents | KF 8018 | Aminopropyl dimethicone | Shin-Etsu Silicone of America | Akron, OH | 7.07 |
| Powder Feel Agents | KSP 100 | Vinyl dimethicone/methicone silsesquioxane crosspolymer | Shin-Etsu Chemical Co. | Tokyo, Japan | 5.05 |
| Powder Feel Agents | Cabosil M5 | Silica | Cabot Corp. | Somerset, NJ | 1.52 |
| Powder Feel Agents | Cabosil TS 610 | Dichlorodimethylsilane | Cabot Corp. | Somerset, NJ | 1.52 |

The ingredients were combined in the order they appear in the table into a glass beaker, stirred with a propeller mixer until the resultant composition was completely uniform. The composition was prepared at room temperature. The CAM of the composition B 1 was measured to be 85.6°.

Compositions E1-E5 of the present invention and comparative compositions C6-C15 were made by combining all of the ingredients from B1 and an additional ester (Repellant Agent) as identified, and in the amount as indicated, in Table 4. The general formulations of such compounds were thus as indicated in Table 3.

Table 3

| Function | Trade Name | INCI Name | E1-E5 | C6-C10 | C11-C15 |
|---|---|---|---|---|---|
| Volatilizing Agent | DC 245 Fluid | Decamethyl-cyclopentasiloxane | 56.0 | 53.737 | 50.909 |
| Powder Feel Agents | USG-103 | Dimethicone/vinyl dimethicone crosspolymer | 18.0 | 17.273 | 16.364 |
| Bulking Agents | DC 200 Fluid, 350 cSt | Dimethicone | 5.0 | 4.798 | 4.545 |
| Bulking Agents | KF 8018 | Aminopropyl dimethicone | 7.0 | 6.717 | 6.364 |
| Powder Feel Agents | KSP 100 | Vinyl dimethicone/ methicone silsesquioxane crosspolymer | 10.0 | 9.596 | 9.091 |
| Powder Feel Agents | Cabosil M5 | Silica | 1.5 | 1.439 | 1.364 |
| Powder Feel Agents | Cabosil TS 610 | Dichlorodimethyl-silane | 1.5 | 1.439 | 1.364 |
| Repellant Agent from Table 4 | - | - | 1.0 | 5.0 | 10.0 |

Compositions E1-E5 and C6-C15 were made as follows: the ratios of materials specified in Table 4 were measured out such that the total mixture weight was 100 grams. The Repellant Agents (esters) were added first to the Volatilizing Agents and mixed in a beaker using a propeller mixing blade at 100 RPM for 1-2 minutes. Powder Feel Agents were then added and mixed at 400-500 RPM until there were no visible clumps. CAM was measured for each resultant composition and reported in Table 4.

Table 4

| Example | Repellant Agent | Concentration | CAM |
|---|---|---|---|
| B1 | Base Formulation | - | 85.6° |
| E1 | Octyl isononanoate | 1 | 93.6 |
| E2 | HallStar IPP | 1 | 93.6 |
| E3 | HallStar BST | 1 | 93.8 |

(continued)

| Example | Repellant Agent | Concentration | CAM |
|---|---|---|---|
| E4 | Ceraphyl (DIPA) | 1 | 93.2 |
| E5 | TISC Ester | 1 | 95.2 |
| C6 | Octyl isononanoate | 5 | 82.0 |
| C7 | HallStar IPP | 5 | 82.9 |
| C8 | HallStar BST | 5 | 83.4 |
| C9 | Ceraphyl (DIPA) | 5 | 84.9 |
| C10 | TISC Ester | 5 | 86.7 |
| C11 | Octyl isononanoate | 10 | 59.3 |
| C12 | HallStar IPP | 10 | 73.5 |
| C13 | HallStar BST | 10 | 79.6 |
| C14 | Ceraphyl (DIPA) | 10 | 80.4 |
| C15 | TISC Ester | 10 | 82.4 |

As illustrated in Table 4, adding less than 5% of the Repellant Agent to the base formulation resulted in a composition having a CAM over 90°. This is a significant and surprising increase over the CAM of the base formulation (B1) or the Repellant Agents alone (see Table 1).

Example 2

Several compositions of the claimed invention (E6-E9) and comparative compositions (C16-C27) were made and the CAM of each tested and reported in Table 5. Each of compositions E6-E9 and C16-C27 were made up of three components: the Volatilizing Agent, Powder Feel Agent, and Repellant Agent (Duck Oil (HallStar Octyl Isononanoate)) as identified, and in the amounts as listed, in Table 5. Such compositions were made as follows: the Repellant Agent was added first to the Volatilizing Agent and mixed in a beaker using a propeller mixing blade at 100 RPM for 1-2 minutes. The Powder Feel Agent was then added and mixed at 400-500 RPM until there were no visible clumps.

Table 5

| Comp. | Volatilizing Agent (VA) | INCI | VA Conc . w/w % | Powder Feel Agent (PFA) | PFA Conc . w/w % | RA (Duck Oil - Hallstar) Conc. w/w% | CAM |
|---|---|---|---|---|---|---|---|
| C16 | IDD (Isododecane) | Isododecane | 41.5 | USG 103 | 57.5 | 1 | 78.3° |
| C17 | IDD | Isododecane | 75 | KSP 100 | 24 | 1 | 48.1 |
| C18 | IDD | Isododecane | 91 | TS 610 | 8 | 1 | 83.9 |
| C19 | IDD | Isododecane | 94 | M5 | 5 | 1 | 12.6 |
| C20 | IHD (Isohexadecane) | Isohexadecane | 41.5 | USG 103 | 57.5 | 1 | 77.9 |
| C21 | IHD | Isohexadecane | 75 | KSP 100 | 24 | 1 | 51.2 |
| C22 | IHD | Isohexadecane | 91 | TS 610 | 8 | 1 | 81.4 |
| C23 | IHD | Isohexadecane | 94 | M5 | 5 | 1 | 14.2 |
| C24 | DC 200 (DC 200 5 cST) | Dimethicone | 41.5 | USG 103 | 57.5 | 1 | 71.3 |
| C25 | DC 200 | Dimethicone | 75 | KSP 100 | 24 | 1 | 54.5 |

(continued)

| Comp. | Volatilizing Agent (VA) | INCI | VA Conc . w/w % | Powder Feel Agent (PFA) | PFA Conc . w/w % | RA (Duck Oil - Hallstar) Conc. w/w% | CAM |
|---|---|---|---|---|---|---|---|
| C26 | DC 200 | Dimethicone | 91 | TS 610 | 8 | 1 | 85.1 |
| C27 | DC 200 | Dimethicone | 94 | M5 | 5 | 1 | 21.3 |
| E6 | DC 245 | Decamethyl-cyclopenta-siloxane | 41.5 | USG 103 | 57.5 | 1 | 92.8 |
| E7 | DC 245 | Decamethyl-cyclopenta-siloxane | 75 | KSP 100 | 24 | 1 | 90.5 |
| E8 | DC 245 | Decamethyl-cyclopenta-siloxane | 91 | TS 610 | 8 | 1 | 96.4 |
| E9 | DC 245 | Decamethyl-cyclopenta-siloxane | 94 | M5 | 5 | 1 | 34.1 |

As illustrated in Table 5, the compositions comprising a volatile cyclic silicone carrier, ester, and certain powder feel agents tended to exhibit significantly higher CAM than comparative compositions. In particular, the compositions of the invention exhibited CAM values above 90°.

<u>Example 3</u>

Four compositions of the claimed invention (E10-E13) and a comparative composition (C28) were made and the CAM of each tested and reported in Table 6. Each of the compositions were made up of three components: the Volatilizing Agent, Powder Feel Agent, and Repellant Agent (Duck Oil) as identified, and in the amounts as listed, in Table 6. The compositions were made in the same manner as E6-E9.

Table 6

| Comp. | DC 245 Conc. w/w% | Duck Oil - Hallstar Conc. w/w% | Powder Feel Agent | Characteristics | Conc. | CAM (degrees) |
|---|---|---|---|---|---|---|
| E10 | 41.5 | 1 | USG 103 | High MW silicone elastomer/cross-linked polymer gel in volatile cyclic silicone solvent | 57.5 | 92.8° |
| E11 | 34 | 1 | USG 103 | High MW silicone elastomer/cross-linked polymer gel in volatile cyclic silicone solvent | 65 | 93.8 |

(continued)

| Comp. | DC 245 Conc. w/w% | Duck Oil - Hallstar Conc. w/w% | Powder Feel Agent | Characteristics | Conc. | CAM (degrees) |
|---|---|---|---|---|---|---|
| E12 | 75 | 1 | KSP 100 | High MW silicone cross-linked polymer powder, thickener | 24 | 90.5 |
| E13 | 91 | 1 | TS 620 | Low MW hydrophobic silica, thickener | 8 | 96.4 |
| C28 | 91-94 | 1 | M5 | Low MW untreated silica, thickener | 5-8 | Below 90 |

As illustrated in Table 6, compositions comprising a volatile cyclic silicone carrier, ester, and certain powder feel agents tended to exhibit significantly higher CAM than comparative compositions. In particular, the compositions of the invention exhibited CAM values above 90°.

<u>Example 4</u>

Six compositions of the claimed invention (E14-E19) and seven comparative compositions (C29-C35) were made and the CAM of each tested and reported in Table 7. Each of the compositions were made up of three components: the Volatilizing Agent (DC 245, decamethylcyclopentasiloxane), Powder Feel Agent, and Repellant Agent (Duck Oil - Hallstar) as identified, and in the amounts as listed, in Table 7. The compositions were made in the same manner as E6-E9.

Table 7

| Comp. | DC 245 Conc. w/w% | Duck Oil (Conc. w/w%) | Powder Feel Agent | Powder Feel Agent (Conc. w/w%) | CAM |
|---|---|---|---|---|---|
| E14 | 91 | 1 | TS 610 | 8 | 96.4 |
| E15 | 41.5 | 1 | USG 103 | 57.5 | 92.8 |
| E16 | 75 | 1 | KSP 100 | 24 | 90.5 |
| C29 | 94 | 1 | M5 | 5 | 34.1 |
| E17 | 89 | 3 | TS 610 | 8 | 93.2 |
| E18 | 39.5 | 3 | USG 103 | 57.5 | 90.1 |
| C30 | 73 | 3 | KSP 100 | 24 | 76.6 |
| C31 | 92 | 3 | M5 | 5 | 31.8 |
| E19 | 88 | 4 | TS 610 | 8 | 90.4 |
| C32 | 38.5 | 4 | USG 103 | 57.5 | 83.4 |
| C33 | 72 | 4 | KSP 100 | 24 | 53.4 |
| C34 | 91 | 4 | M5 | 5 | 32.1 |
| C35 | 74 | 2 | KSP | 24 | 86.7 |

As illustrated in Table 7, compositions comprising a volatile cyclic silicone carrier, ester, and certain powder feel agents tended to exhibit significantly higher CAM than comparative compositions. In particular, the compositions of the invention exhibited CAM values above 90°.

Example 5

The Body Dryness Index for the base formulation B1 and Examples E1-E5 was measured in accord with the procedure below and is reported in Table 8.

**Test Procedure for Body Dryness Index**

Test fluid was made of the following mixture to simulate bodily fluids: 49.5% of 0.9% sodium chloride solution (VWR catalog # VW 3257-7), 49.05% Glycerin (Emery 917), 1% Phenoxyethanol (Clariant Corporation Phenoxetol.TM.) and 0.45% Sodium Chloride (Baker sodium chloride crystal # 9624-05).

For each test composition (B1, E1-E5), a 2"x2" sample of hydrated V-skin (Vitro-skin-N-19 manufactured by IMS Inc, 70 Robinson Blvd, Orange, CT 06477; Telephone # 203-795-9047) was cut and the weight recorded. This film sample provides a liquid impervious surface and represents the body in this test. The test composition, 0.5 grams thereof, is spread uniformly across the top surface of the film to form a test sample (Gardner Draw with variable thickener was used with 1.5mil set clearance is used to spread the test composition evenly across the hydrated V-skin to form a substantially uniform coated film on the V-skin). A control sample with no test composition applied thereto was prepared. All samples were weighed and recorded as initial weights. N=3

Using a Corning Syringe Pump, 10 mL of test fluid was dispensed onto the surface of the test samples and control sample. The test samples and control sample were then allowed to rest for 2 minutes. Each film was then tilted at a 45 incline for 1 minute to allow excess fluid to drain off. The weights were recorded as final weights N=3.

**Calculating the Body Dryness Index**

The Body Dryness Index (BDI) is based on the Body Wetness Index (BWI), which is the residual fluid on the skin (g) divided by the total weight of the fluid dispensed.

$$\text{Body Wetness Index (BWI)} = \frac{\text{residual fluid on skin (g)}}{\text{Total weight of fluid dispensed (g)}}$$

$$\text{Body Dryness Index (BDI)} = \frac{1}{\text{BWI}}$$

Table 8 represents the volume of test fluid absorbed by the V-skin uncoated or coated with the test composition for certain times. The values were generated by subtracting the initial weight($W_i$) from the fmal weight($W_f$) for each time period.

Table 8

| Sample | $W_f$-$W_i$ | BDI |
|---|---|---|
| Control (uncoated skin) | | 74 |
| B1 | | 1250 |
| E1 | 0.006 | 1667 |
| E2 | 0.007 | 1429 |
| E3 | 0.005 | 2000 |
| E4 | 0.003 | 3333 |
| E5 | 0.003 | 3333 |

As shown by the above data, surfaces coated with a layer of the test composition containing a repellent agent

had a higher BDI that the surface coated with just the control test formulation (no repellent agent).

Personal Product Kit

According to another aspect of the invention, the present invention provides a personal product kit including in combination a product dispenser assembly as described above, a composition of the type described above provided within the product dispenser assembly, and at least one absorbent article. As used herein the term "absorbent articles" includes articles such as diapers (infant and adult), sanitary napkins, shields, pantyliners, breast pads, and the like.

A specific example of a personal product kit according to the present invention could include, a product dispenser assembly as described above, a composition of the type described above adapted to be applied the vaginal area of a user's body, and at least one sanitary napkin. To use such a kit, a user would apply the composition to vaginal area using the dispenser assembly and then wear the sanitary napkin in a conventional fashion. The use of the composition in this manner would promote menstrual and/or urine flow into the napkin and thereby provide a clean dry feeling to the user. Kits according to the present invention could of course be provided with a plurality of absorbent articles, e.g. a plurality of sanitary napkins, to enable a user to replace the used absorbent after it has been soiled. Kits according to the present invention may alternative include other absorbent articles, such as pantyliners or diapers, in combination with a product dispenser assembly and a composition of the type described above.

Another specific example of a personal product kit according to the present invention could include, a product dispenser assembly as described above, a composition of the type described above adapted to be applied the nipple area of a user's body, and at least one breast pad. To use such a kit, a user would apply the composition to nipple area using the dispenser assembly and then wear the breast pad. The use of the composition in this manner would promote the flow of fluid into the breast and thereby provide a clean dry feeling to the user.

## Claims

**1.** A product dispenser assembly comprising:

a container housing including an internal chamber for holding a composition and a head portion, the head portion including at least one aperture, the container housing being structured and arranged to enable a user to selectively deploy the composition from within the internal chamber through the at least one aperture;
a cap assembly structured and arranged to be placed on top of the head portion of
the container, the cap assembly including an outer shell having a first open end and a second closed end;
a first absorbent removable pad arranged on top of the head portion for receiving the composition as it is deployed from the container, the first removable pad being adapted to enable a user to apply the composition to the body and manually remove the pad from the head portion after the composition has been applied;
a plurality of absorbent pads arranged in a stacked configuration within the outer shell, each of the plurality of pads being adapted to be sequentially arranged on top of the head portion after the first removable pad has been used to apply the composition and then removed from the head portion by the user.

**2.** A product dispenser assembly according to claim 1 wherein the head portion includes a plurality of apertures.

**3.** A product dispenser assembly according to claim 1 or claim 2 wherein the container housing comprises a moveable member arranged within the container housing that is selectively moveable by a user within the container to allow a user to selectively manipulate a volume of the internal chamber, the moveable member being adapted to enable a user to selectively deploy the composition from within the internal chamber, through the at least one aperture, by reducing the volume within the chamber.

**4.** The product dispenser assembly according to any preceding claim, wherein the plurality of pads includes a lowermost pad and a plurality of pads arranged on top of the lowermost pad.

**5.** The product dispenser assembly according to claim 4, wherein the lowermost pad is adapted to be arranged on top of the head portion after the first removable pad has been used to apply the composition to a body of a user and then removed by the user from the head portion of the assembly.

**6.** The product dispenser assembly according to claim 5, wherein the lowermost pad is automatically applied to, and retained on, the head portion by placing the cap assembly on top of the head portion of the container housing.

**7.** The product dispenser assembly according to any preceding claim, further comprising:

a platform arranged within the outer shell, the platform being structured and arranged to urge the plurality of pads toward the open end of the outer shell.

**8.** The product dispenser assembly according to claim 7, further comprising:

a member arranged between the platform and the outer shell, the member being adapted to urge the platform towards the open end of the outer shell.

**9.** The product dispenser assembly according to any preceding claim, further comprising a material arranged on a top surface of the head portion, the material 5 including a plurality of upwardly extending hooks, the hooks being adapted to retain the removable pad against the head portion during application of the composition to the body.

**10.** The product dispenser assembly according to any preceding claim, wherein the outer shell includes a rib structure structured and arranged to retain the plurality of pads within the outer shell.

**11.** The product dispenser assembly according to 5, wherein the lowermost pad is structured and arranged to be manually removed from the outer shell and manually applied to the top of the head portion by a user.

**12.** The product dispenser assembly according to claim 11, wherein the outer shell is provided with an access port to enable a user to manually remove the lowermost pad from within the outer shell.

**13.** The product dispenser assembly according to claim 3, wherein the moveable member is coupled to an elevator screw structured and arranged to enable a user selectively control a movement of the moveable member.

**14.** The product dispenser assembly according to claim 13, wherein the moveable member is coupled to the elevator screw by means of a threaded coupling sleeve.

**15.** The product dispenser assembly according to any preceding claim, wherein the composition comprises a volatile cyclic silicone carrier, a silicone-based powder-feel agent, and less than 5 weight % of an ester selected from the group consisting of formula I, formula II, formula III, and combinations of two or more thereof:

O

||

$R_1$-C-O-$R_2$ (I)

O O

|| ||

$R_3$- C- O - $R_4$- O - C- O – $R_5$ (II)

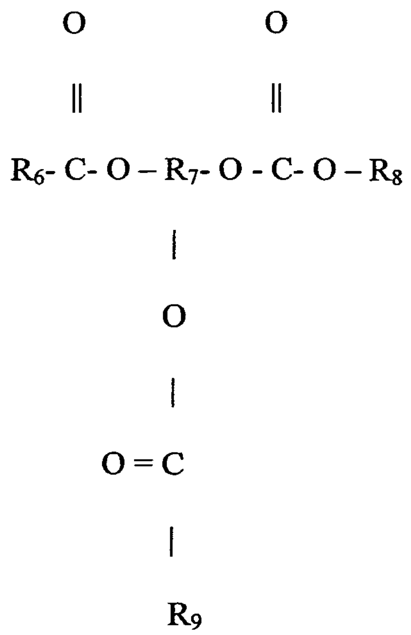

(III)

wherein $R_1$, $R_2$, $R_3$, $R_5$, $R_6$, $R_8$ and $R_9$ are independently linear or branched, substituted or unsubstituted, saturated or unsaturated, $C_3$-$C_{22}$ alkyl or alkenyl groups, $R_4$ is a linear or branched, substituted or unsubstituted, saturated or unsaturated, $C_3$-$C_{22}$ alkylene or alkenylene moiety, and $R_7$ is a linear or branched, substituted or unsubstituted, saturated or unsaturated $C_3$-$C_{22}$ moiety, the composition being substantially anhydrous.

**16.** The product dispenser assembly according to claim 15, wherein the composition has a Contact Angle of 90° or greater.

**17.** The product dispenser assembly according to claim 15 or claim 16, wherein said cyclic volatile silicone carrier is a cyclomethicone; or wherein said cyclic volatile silicone carrier is selected from decamethylcyclopentasiloxane and dodecamethylcyclohexasiloxane; or is decamethylcyclopentasiloxane.

**18.** The product dispenser assembly according to any one of claims 15 to 17, wherein said powder feel agent is selected from the group consisting of silicone polymers, silicone gels, silicone gums, hydrophobic silica particles, and combinations of two or more thereof; or wherein said powder feel agent is selected from the group consisting of high-molecular weight crosslinked silicone polymers, high molecular weight elastomer crosslinked silicone polymer gels, hydrophobic silica particles, and combinations of two or more thereof; or wherein the powder feel agent comprises a high molecular weight cross-linked silicone elastomer; or wherein the powder feel agent comprises a hydrophobic silica particle.

**19.** The product dispenser assembly according to any one of claims 15 to 17, wherein the ester comprises an unbranched monoester; or a branched monoester; or a diester; or a triester; or wherein the ester is selected from the group consisting of hexyldecyl benzoate, hexyl laurate, hexadecyl isostearate, hexydecyl laurate, hexyldecyl octanoate, hexyldecyl oleate, hexyldecyl palmitate, hexyldecyl stearate, hexyldodecyl salicylate, hexyl isostearate, butyl acetate, butyl isostearate, butyl oleate, butyl octyl oleate, cetyl palmitate, cetyl octanoate, cetyl laurate, cetyl lactate, octyl isononanoate, isostearyl isononanoate, isononyl isononanoate, cetyl isononanoate, cetyl stearate, stearyl lactate, stearyl octanoate, stearyl heptanoate, stearyl stearate, and combinations of two or more thereof; or wherein the ester is selected from the group consisting of octyl isononanoate, isopropyl palmitate, butyl stearate, diisopropyl adipate, triisostearyl citrate, and combinations of two or more thereof; or wherein the ester is selected from the group consisting of octyl isononanoate, isopropyl palmitate, butyl stearate, and combinations of two or more thereof; or wherein the ester is octyl isononanoate.

**20.** The product dispenser assembly according to claim 15 or claim 16, wherein said volatile cyclic silicone carrier

comprises decamethylcyclopentasiloxane, said silicone-based powder-feel agent comprises a silicone elastomer blend, hydrophobic silica, or a combination thereof, and said ester comprises octyl isononanoate, isopropyl palmitate, butyl stearate, diisopropyl adipate, triisostearyl citrate, or a combination of two or more thereof.

**21.** A kit comprising:

a product dispenser assembly according to any one of claims 1 to 20; and
at least one absorbent article.

**22.** The kit according to claim 21, wherein the absorbent article is selected from a sanitary napkin, a pantiliner, a diaper and a breast pad.

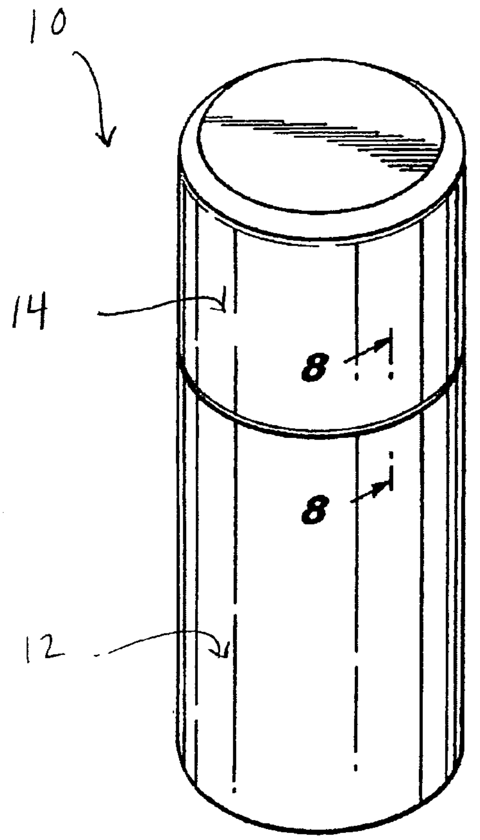


Fig. 1

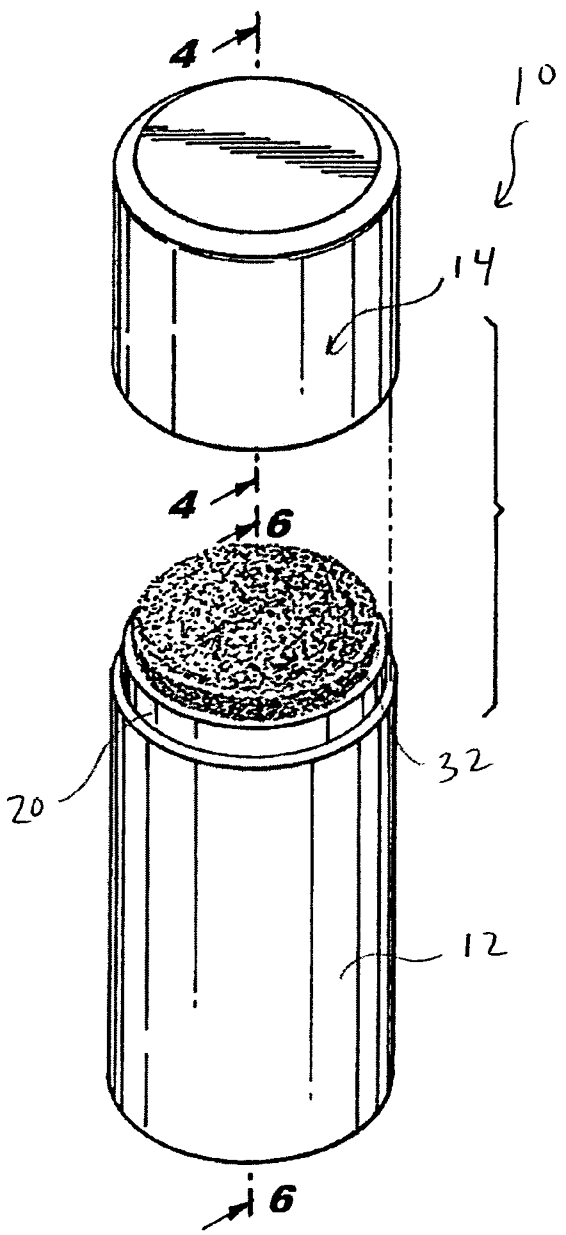


Fig. 2

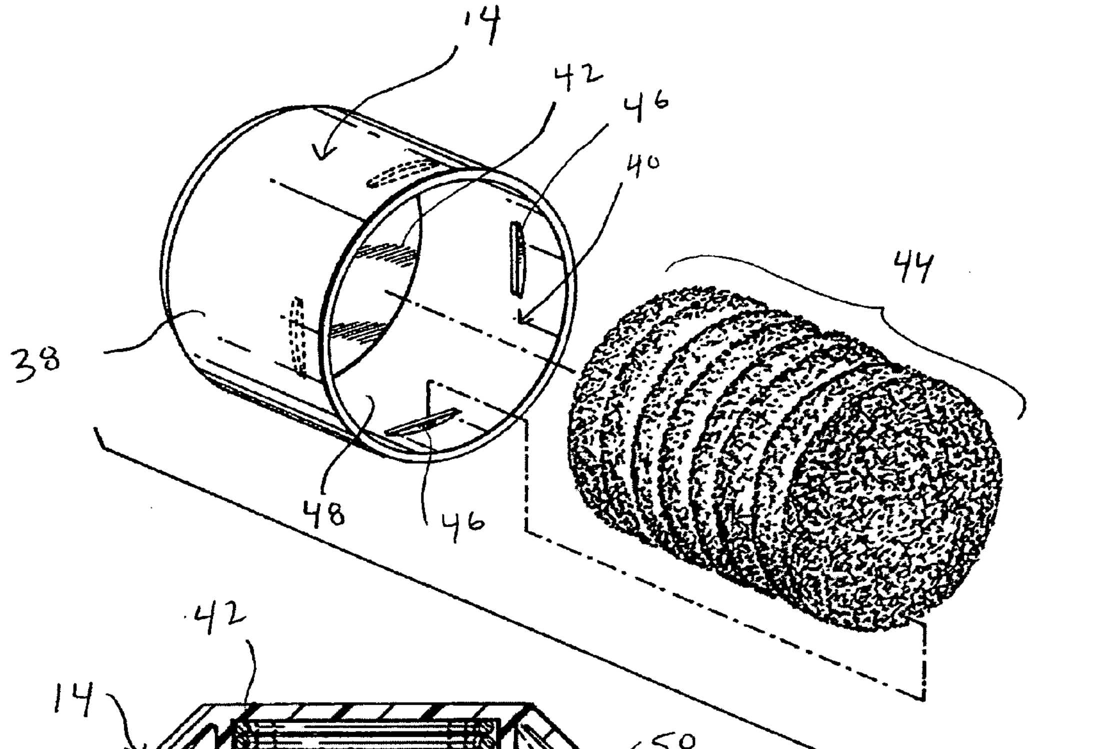


Fig. 3

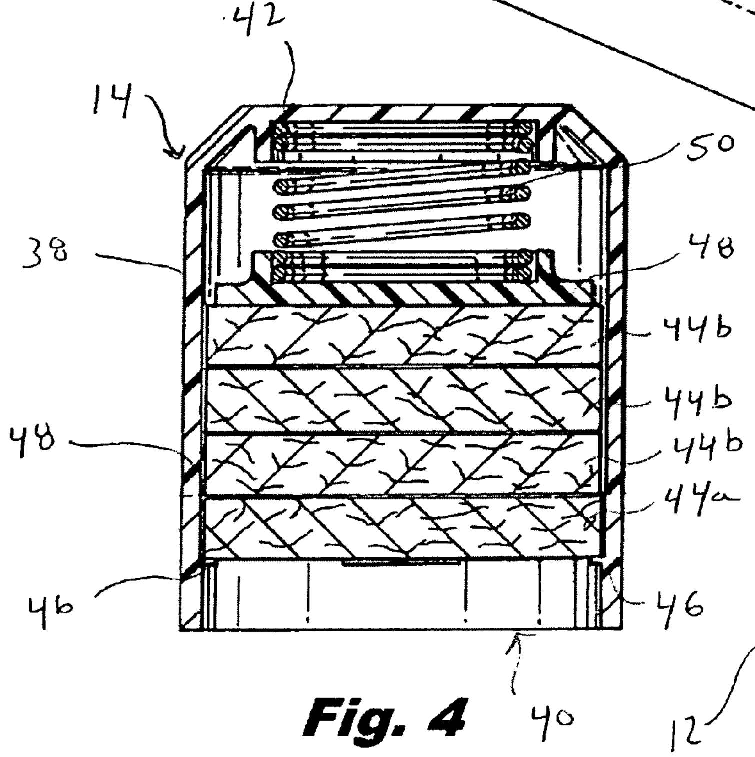


Fig. 4

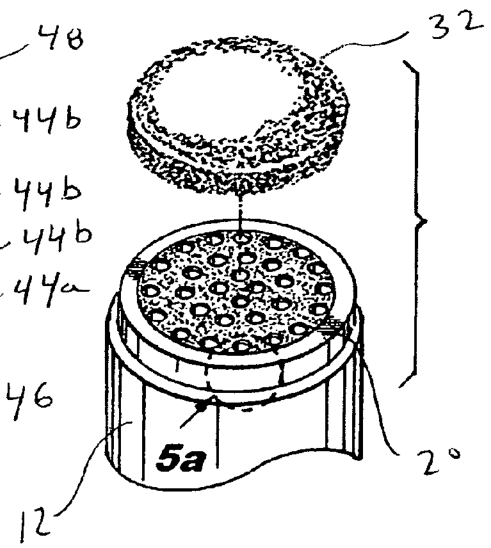


Fig. 5

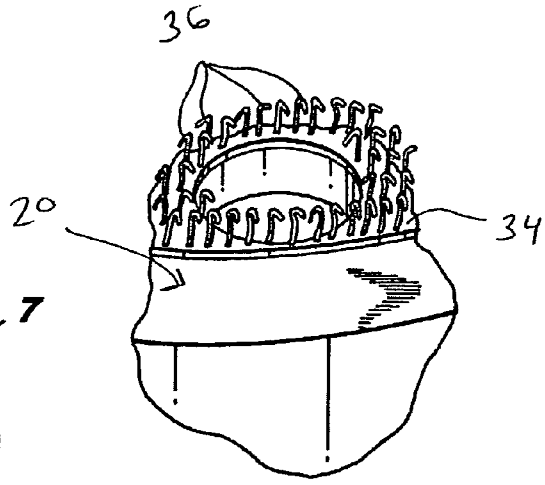


**Fig. 5a**

**Fig. 6**

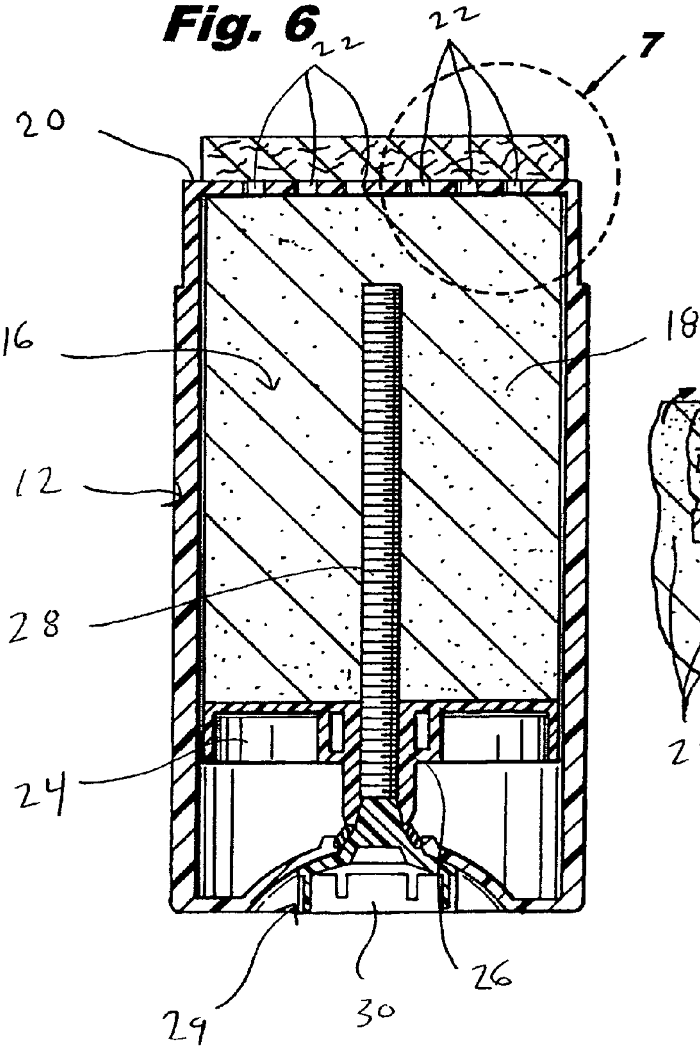


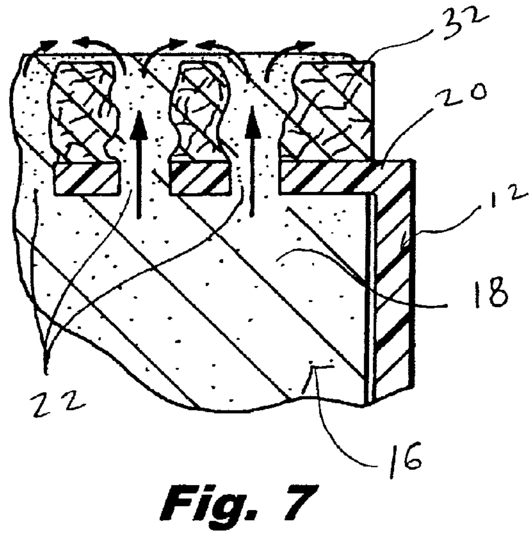


**Fig. 7**

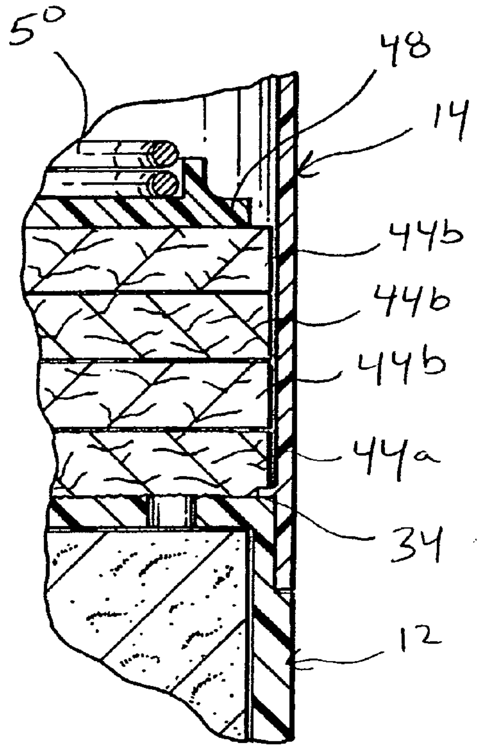
50
48
14
44b
44b
44b
44a
34
12

**Fig. 8**

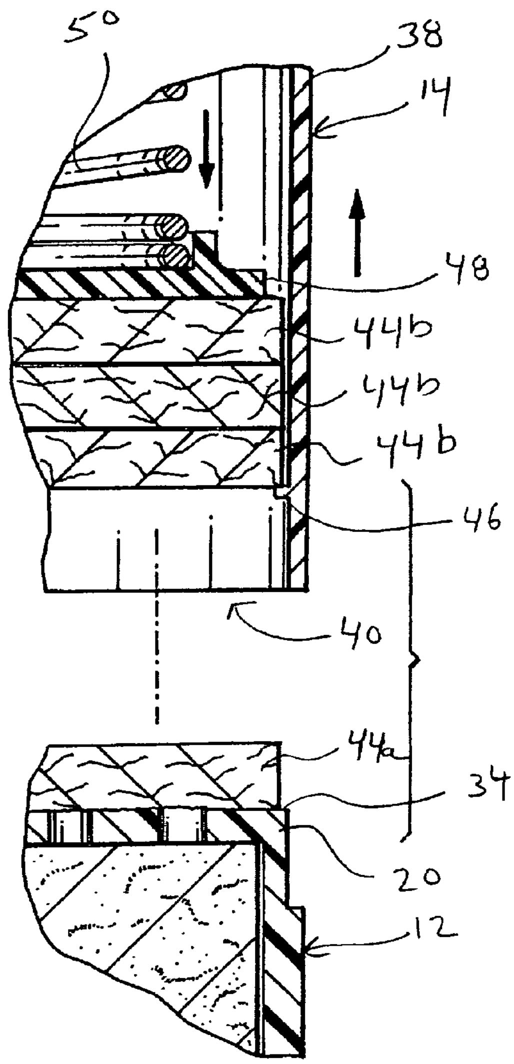
50
38
14
48
44b
44b
44b
46
40
44a
34
20
12

**Fig. 9**

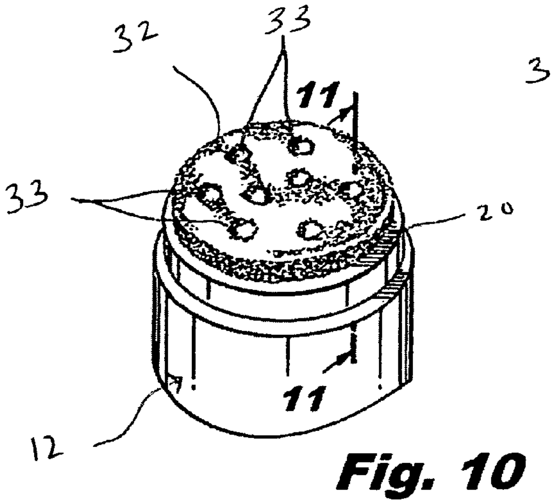


Fig. 10

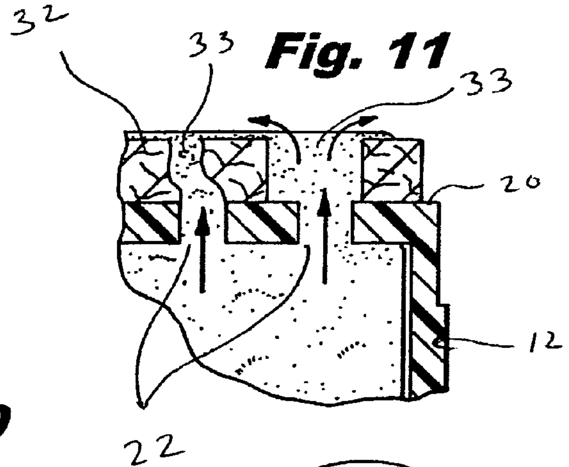


Fig. 11

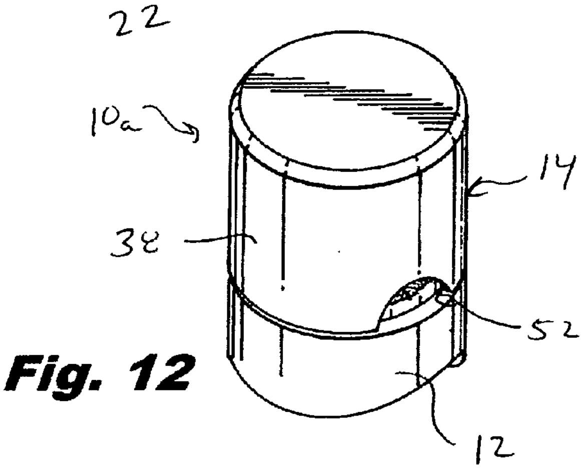


Fig. 12

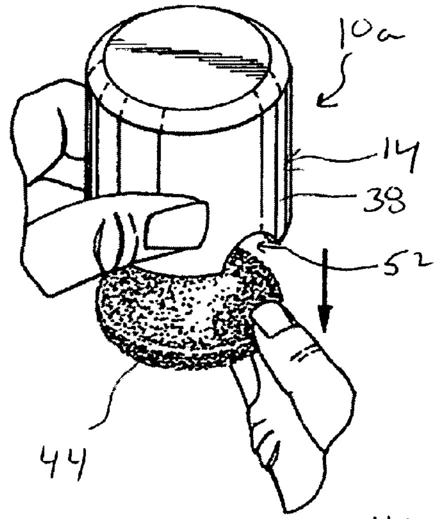


Fig. 13

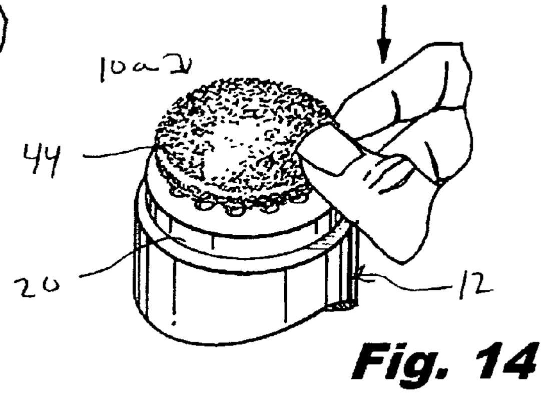


Fig. 14

Europäisches Patentamt
European Patent Office
Office européen des brevets

# EUROPEAN SEARCH REPORT

Application Number
EP 10 25 2138

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 2 699 780 A (RUDNICK JOSEPH B ET AL) 18 January 1955 (1955-01-18)<br>* column 1, lines 15-18 *<br>* claims 1-3; figures 1-6 * | 1-8, 10-14,21<br><br>15-20,22 | INV.<br>A61F15/00<br>A47F1/12<br>A61K8/25<br>A61K8/58<br>A61K8/895<br>B65D83/04 |
| X<br><br><br>Y | WO 99/48456 A1 (UNILEVER PLC [GB]; UNILEVER NV [NL]; LEVER HINDUSTAN LTD [IN]) 30 September 1999 (1999-09-30)<br>* page 2, lines 19-28 *<br>* page 3, lines 21-24 *<br>* page 5, lines 1-14 * | 1,4,21<br><br><br>15-20 | |
| X,P | EP 2 151 174 A1 (CMC CONSUMER MEDICAL CARE GMBH [DE]) 10 February 2010 (2010-02-10)<br>* page 2, paragraphs 1,6 *<br>* page 3, paragraph 9-11 *<br>* figures 1,2 * | 1,4,21 | |
| Y | US 5 217 641 A (HERSTEIN MORRIS [US]) 8 June 1993 (1993-06-08)<br>* column 4, lines 1-12 * | 15-20 | TECHNICAL FIELDS SEARCHED (IPC) |
| Y | US 3 860 304 A (BOLTON RONALD L) 14 January 1975 (1975-01-14)<br>* column 1, line 24 - column 2, line 63 * | 22 | A61F<br>A47F<br>A61K<br>B65D |

The present search report has been drawn up for all claims

| Place of search | Date of completion of the search | Examiner |
|---|---|---|
| Munich | 24 May 2011 | Beins, Ulrika |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 25 2138

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2011

| Patent document cited in search report | | Publication date | Patent family member(s) | | | Publication date |
|---|---|---|---|---|---|---|
| US 2699780 | A | 18-01-1955 | NONE | | | |
| WO 9948456 | A1 | 30-09-1999 | AU | 736879 | B2 | 02-08-2001 |
| | | | AU | 3143699 | A | 18-10-1999 |
| | | | BR | 9909055 | A | 05-12-2000 |
| | | | CA | 2319469 | A1 | 30-09-1999 |
| | | | CN | 1293557 | A | 02-05-2001 |
| | | | CZ | 20003521 | A3 | 16-05-2001 |
| | | | DE | 69910185 | D1 | 11-09-2003 |
| | | | DE | 69910185 | T2 | 26-02-2004 |
| | | | EP | 1066009 | A1 | 10-01-2001 |
| | | | ES | 2204113 | T3 | 16-04-2004 |
| | | | HK | 1032734 | A1 | 21-01-2004 |
| | | | ID | 26039 | A | 16-11-2000 |
| | | | JP | 2002507556 | T | 12-03-2002 |
| | | | PL | 343074 | A1 | 30-07-2001 |
| | | | RU | 2207108 | C2 | 27-06-2003 |
| | | | TW | 581673 | B | 01-04-2004 |
| | | | ZA | 9900526 | A | 25-07-2000 |
| EP 2151174 | A1 | 10-02-2010 | NONE | | | |
| US 5217641 | A | 08-06-1993 | NONE | | | |
| US 3860304 | A | 14-01-1975 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6200964 B
- US 6384023 B, Singleton
- US 20060159645 A, Miller
- US 20030049212 A1
- US 5412004 A
- US 5837793 A
- US 5811487 A
- US 20030171475 A

### Non-patent literature cited in the description

- International Cosmetic Ingredient Dictionary and Handbook. The Cosmetic, Toiletry, and Fragrance Assoc, 1997